# EUROPEAN PATENT SPECIFICATION

(11) **EP 1 240 897 B1**
(45) Date of publication and mention of the grant of the patent: **21.06.2006**
(21) Application number: 02251649.6
(22) Date of filing: 08.03.2002
(51) Int. Cl.: A61K 9/20, A61K 9/00, A61K 31/495

(54) **Pharmaceutical tablet and process for making thereof**
Pharmazeutische Tablette und eine Verfahren zu deren Herstellung
Comprimé pharmaceutique et son procédé de fabrication

(30) Priority: 14.03.2001 US 275889 P
(43) Date of publication of application: 18.09.2002
(73) Proprietor: Pfizer Products Inc., Groton, Connecticut 06340 (US)
(72) Inventor: Waterman, Kenneth C., c/o Pfizer Global Res. & Dev, Groton, Connecticut 06340 (US)
(74) Representative: Ruddock, Keith Stephen

(56) References cited:
- EP-A- 0 812 589
- WO-A-95/20946
- US-A- 3 121 044
- US-A- 4 892 741
- US-A- 5 738 874
- US-A- 6 004 582
- US-A- 6 136 345

## Description

### FIELD OF INVENTION

The present invention relates to a pharmaceutical tablet and a process for manufacturing thereof, in particular, a tablet wherein a drug having a defined rate of delivery is applied by compression onto a compressible coating that has been deposited on a tablet having the same or a different *in vivo* drug release profile.

### BACKGROUND

Controlled release drug delivery systems involving coatings to control the rate or timing of drug release are used widely to improve drug toleration or to provide easier dosing regiments. Among these technologies are osmotic systems where a large range in delivery rates for drugs of varying solubilities can be achieved (see, e.g., Santus, G. and R. W. Baker, J. Control. Rel., **35**, 1-21 (1995); US Patent Nos. 5,612,059; 5,698,220; and 4,857,331). Enteric coated tablets have been demonstrated to allow one to orally administer a drug that is otherwise unstable in or shows adverse events in the stomach.

European Patent Application No. 0812589 directed to a combined antipyretic analgesic drug, US Patent No. 6,136,345 directed to a tablet containing a coated core, US Patent No. 6,004,582 directed to a multi-layered osmotic device, International Patent Application No. WO 95/20946 directed to bilayered amoxycillin tablets, US Patent No. 5,738,874 directed to a pharmaceutical tablet capable of liberating one or more drugs at different release rates, US Patent No. 4,892,741 directed to press coated DHP (dihydropyridine) tablets and US Patent No. 3,121,044 directed to a three-layer compressed penicillin tablet are all considered relevant background art in respect of the present invention.

A growing interest has developed in designing drug delivery systems that include an immediate release (IR) component to these controlled-release (CR) behaviors. The addition of an IR component allows one to design a delivery system having an optimum pharmacokinetic profile and enables the combination of different drugs thereby improving patient compliance or reducing adverse events associated with one of the drugs.

Several approaches have previously been described and/or tested. For example, U.S. Patent No. 5,338,550 describes a bilayer system where the IR component and CR component are independently subjected to a wet granulation process and then compressed sequentially to form a single tablet. Although this provides a combination of delivery profiles, the CR portion is not coated; therefore, an IR dosage form cannot be combined with an osmotic or enteric controlled release dosage using this process.

CR- or enteric-coated beads and immediate-release beads (or multiparticulates) can be combined in a capsule. However, this approach may be difficult to implement in an osmotic system since controlling delivery with small beads has not been demonstrated to be feasible for most drugs. In some instances, mixing small beads of some drugs can be problematic due to the process involved. It is also difficult to achieve high doses with this approach since there is a greater volume needed for beads in a capsule than for a tablet with the same dose. In addition, there can be packaging and stability issues associated with the capsules.

One could also form a bilayer system by spray-coating a coated core CR tablet with a liquid coating containing the IR drug either as part of the active coating or on the outside of the active coating (see, e.g., US Patent No. 4,576,604). In both of these approaches, the drug must either be dissolved or dispersed in the coating fluid. In many cases, dissolution of the drug results in stability issues either because the drug itself is unstable in solution or because the coated drug is in a less stable amorphous state. Although drug dispersions can be prepared and coated, many drugs present difficult challenges. For example, the drug must be very insoluble in the coating solvent. The drug must also be kept in suspension during the spray-coating process. Content uniformity (tablet to tablet) can be especially challenging if there is settling or agglomeration. Even from solution, tablet-coating processes can lead to unacceptable variability in potency. In addition, environmental concerns favor the use of water as the solvent for coating; however, for many drugs, aqueous coating is not ideal. Another challenge associated with coating a drug onto the surface is the difficulty in adding disintegrants to the formulation. A disintegrant may be necessary for rapid dispersion of the drug in the GI tract and to allow for rapid dissolution and absorption.

Another approach for delivering two profiles for drugs involves the use of a bilayer osmotic system where two active layers have different dissolution profiles and are therefore delivered at different rates. This method is in general incompatible with the principles of osmotic delivery where rates are determined predominantly by the water flux into the core thus requiring specific drug/excipient development to be useful.

In yet another approach, an immediate release solid dosage coating can be compressed around a coated tablet (see, e.g., U.S. Patent No. 6,136,345). This approach has the advantage that the IR dosage is relatively standard in form; however, there are difficulties with this approach as well. First, the quantity of material required to surround the entire tablet is significant thus limiting the applicability of this technology. In fact, the surrounding compression coat, in general, must be larger than the core itself. Since many osmotic and enteric systems already have a significant amount of excipient, the final tablet size after compression coating can become prohibitive. Second, since both the osmotic and enteric tablet coatings are relatively hard, there is essentially no compressibility. As a result, the compressed shell adheres poorly to the controlled-release tablet which can lead to physical instability (i.e., friability). Third, since the coated tablets that form the core in this approach will often have significant dimensional variability, standard powder filling will lead to potency variability in the second active drug. This issue is especially problematic when the second drug is added in relatively concentrated form to minimize the increase in core tablet dimensions and even more troublesome for thicker osmotic or enteric film coatings since the core dimensional variability can be increased significantly due to the film coating variability.

Controlled release coatings in use today have almost exclusively been made from cellulosics. In particular, cellulose acetate and ethyl cellulose have been used to produce a number of systems. Additional systems have involved hydroxypropylmethyl cellulose and other cellulose derivatives. These coatings have been plasticized by such additives as polyethylene glycol (PEG) and triacetin (also known as glycerol triacetate available from Sigma Chemical Co., St. Louis, MO). In all of these cases, the membrane is a hard material with high yield pressures and limited elasticity. The resulting tablet upon eventual defecation (depending on the coating thickness) can appear to be a solid, intact, tablet. This can result in patient concern over the drug delivery and proper performance of the dosage form.

In summary, there is a need for a practical method for adding an IR dosage form to an osmotic CR or enteric delivery system that can be applied to a wide range of drugs. To avoid the formation of an amorphous form of the drug, the process needs to allow for the combination of two actives without the dissolution in a liquid or solvent coating. There is also a need for a dosage form that contains low to moderate amounts of IR drug without adding significantly to the tablet volume. In addition, the technology and materials used must be acceptable to the pharmaceutical industry and the controlling regulatory agencies.

### SUMMARY

The present invention provides a process for compressing a drug powder onto a coated tablet and the tablet generated therefrom. In summary, a pharmaceutical tablet is provided which includes a pharmaceutical core containing a placebo or a first drug and having a top side, a bottom side and edges, a compressible coating (preferably water-permeable) deposited on the core and a second drug compressed onto the compressible coating adjacent to the bottom side of the core to form a first compressed layer. Although references are made to a top side, bottom side and edges, the references are used for general points of reference and not indicative of any order in which the powder(s) is applied to the compressible coating nor indicative of any particular shape of the pharmaceutical core or tablet. The tablet may optionally contain a placebo or a third drug compressed onto the compressible coating adjacent to the top side and optionally the edges of the core to form a second compressed layer. The weight ratio of the combined first compressed layer and second compressed layer to the pharmaceutical core is preferably from about 1:20 to less than about 1.25:1, more preferably from about 1:10 to about 1:1, and most preferably from about 1:5 to about 9:10.

The pharmaceutical core may be a single or multi-layered construction and may have a predetermined drug release profile (e.g., an osmotic controlled release core or a pharmaceutically active core having deposited thereon an enteric coating). The compressible coating preferably comprises a gum-based resin (e.g., polyvinyl acetate resin, preferably a polyvinylacetate having a weight average molecular weight from about 2,000 to about 20,000, more preferably from about 10,000 to about 15,000) and a plasticizer. The plasticizer is preferably a water-soluble plasticizer (e.g., polyethyleneglycol). The compressible coating may also function as an enteric or an osmotic coating. The second drug may be formulated for immediate release or controlled release. The first drug (if present), the second drug and third drug (if present) may all be the same, each different, or two the same and one different. In a preferred embodiment, the first drug is pseudoephedrine and the second drug is cetirizine. The drug release profiles for the first drug (if present), the second drug and the third drug (if present) may be all the same, each different, or two the same and one different.

In another embodiment of the present invention, a pharmaceutical tablet is provided that includes a pharmaceutically active core having deposited thereon a compressible coating, wherein the compressible coating functions as an osmotic controlled release coating. The controlled release coating can function as a semi-permeable membrane thus allowing it to be used as an osmotic coating, or as a permeable coating for use with coated matrix-type controlled release systems. Additional coatings may be applied over the compressible coating (e.g., coatings to provide protection, flavor enhancement, printable surface, etc.).

In yet another embodiment of the present invention, a process is provided for manufacturing a pharmaceutical tablet that includes the steps of:
(i) providing a pharmaceutical core containing a placebo or a first drug and having deposited thereon a compressible coating (or layer, preferably water-permeable);
(ii) placing a first powder comprising a second drug into a die press;
(iii) placing the pharmaceutical core from step (i) in intimate contact with the first powder in the die press;
(iv) compressing the first powder onto the compressible coating on the core to form a compressed tablet; and
(v) optionally, coating the compressed tablet from step (iv) with an outer coating.
The second drug may be formulated to be released at a faster rate than the first drug. (e.g., The second drug has an immediate release profile and the first drug has a controlled release profile.) Alternatively, the drug release profile of the second drug may be the same as the first drug. The compressible coating preferably comprises a gum-based resin (e.g., polyvinyl acetate resin, preferably a polyvinylacetate having a weight average molecular weight from about 2,000 to about 20,000, more preferably from about 10,000 to about 15,000) and a plasticizer. The plasticizer is preferably a water-soluble plasticizer (e.g., polyethyleneglycol). The compressible coating may also function as a controlled release coating. The first drug and the second drug may be the same or different. In a preferred embodiment, the first drug is pseudoephedrine and the second drug is cetirizine.

In yet another embodiment of the present invention, a process for manufacturing a pharmaceutical tablet is provided which includes the steps of:
(i) providing a pharmaceutical core containing a placebo or a first drug and having deposited thereon a compressible coating (or layer, preferably water-permeable);
(ii) placing a first powder into a die press;
(iii) placing the pharmaceutical core from step (i) in intimate contact with the first powder in the die press;
(iv) placing a second powder (optionally containing a third drug) in intimate contact with the pharmaceutical core on the opposite side from the first powder in the die press;
(v) compressing both the first powder and the second powder onto the compressible coating on the core to form a compressed tablet; and
(vi) optionally, coating the compressed tablet from step (v) with an outer coating;
wherein either said first powder or said second powder comprises a second drug. The first drug (if present), second drug and third drug (if present) may each have a different delivery rate, or the third drug (if present) may have a different rate of delivery from the first or second drug, or the third drug (if present) may have the same delivery rate as the first (if present) or second drug. The rate of delivery of the first and second drugs may be the same or different. The compressible coating preferably comprises a gum-based resin (e.g., a polyvinyl acetate resin, preferably a polyvinylacetate having a weight average molecular weight from about 2,000 to about 20,000, more preferably from about 10,000 to about 15,000) and a plasticizer. The plasticizer is preferably a water-soluble plasticizer (e.g., polyethyleneglycol). The compressible coating may also function as a controlled release coating.

A pharmaceutical tablet prepared by any one of the methods described above is also provided.

### Definitions

As used herein, the term "compressible coating" refers to a coating having a yield pressure that is preferably less than or equal to about 350 MegaPascals (MPa), more preferably less than or equal to about 100 MPa and most preferably, less than or equal to about 50 MPa. For a detailed description, see, E. N. Hiestand, J. M. Bane, Jr. and E. P. Strzelinski, "Impact test for hardness of compressed powder compacts", *J. Pharm. Sci.,* **60** (5), pp. 758-763 (1971).

The term "drug" refers to a pharmaceutically active ingredient(s) or prodrug thereof and any pharmaceutical composition formulated to elicit a therapeutic effect. Pharmaceutical compositions include formulations as well as dosage forms or medicaments (e.g., powders, capsules and tablets).

The term "pharmaceutical core" refers to a tablet core containing pharmaceutically acceptable excipients, diluents and/or carriers formed into a single uniform solid (i.e., single layer), or a multi-layered solid (e.g., compressed multi-layer construction, coated compressed core, or combination thereof). The additional coatings or layers may be present for a variety of functions (e.g., controlled-release, enteric-release, delayed-release, adhesion enhancement of adjacent coatings or layers, identification (e.g., trademark), taste masking, to add drug combinations and for protection from environmental elements such as light, moisture and/or oxygen. The core may contain a drug (referred to herein as a "pharmaceutically active core") or a placebo.

The term "controlled release" refers to a dosage form designed to meter a drug into a use environment such that the drug is available for absorption over a period of time greater than one hour.

The term "immediate release" refers to a dosage form designed to allow the majority of a drug to be made available for absorption in a period of time less than about one hour.

The term "osmotic controlled release" refers to a controlled release dosage form where the means for metering the drug is controlled by osmotically driven ingress of water through a membrane.

The term "delayed release" refers to a dosage form designed to release drug rapidly or in a controlled release fashion where the delivery of the drug does not begin until either a predetermined time has passed or the dosage form reaches a certain environment during its transit through the GI tract.

The term "enteric release" refers to drug delivery systems designed to deliver drug in the intestine with little or no delivery of the drug in the stomach.

### BRIEF DESCRIPTION OF THE DRAWINGS

Figure 1 illustrates the general processes for adding a drug powder onto a coated controlled release (CR) system according to the present invention. The figure is drawn to illustrate the general process and should not be construed as representing any relative dimensions or particular shapes of the core and/or tablet.
Figure 2 is a graph showing the dissolution curve of the pseudoephedrine tablets from Example 1. The control is the gum coated pseudoephedrine tablet. The test sample is the gum coated pseudoephedrine tablet compressed with cetirizine.
Figure 3 is a graph showing the dissolution rates for a control versus tablets containing both an IR layer and placebo layer compressed onto CR tablets from Example 2.

### DETAILED DESCRIPTION

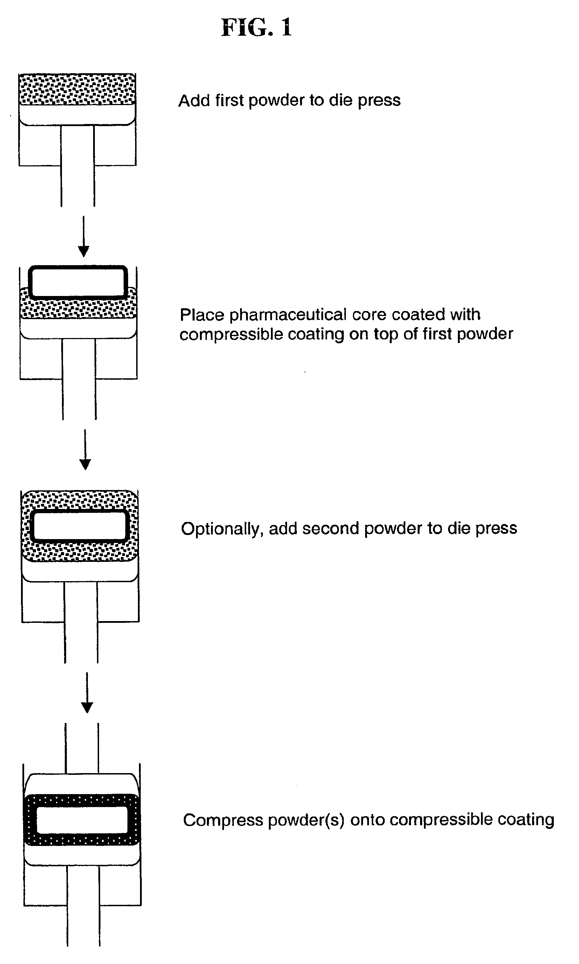

Figure 1 illustrates the general process for adding a drug powder (e.g., active blend or granulation) onto a coated CR system according to the present invention. A pharmaceutical core (single layer or multi-layered core) is coated with a thin layer of a film that can yield under pressure (i.e., low yield point). By yielding under pressure, it is meant that the thin film coating allows powders to adhere to the tablet under compression conditions. A powder blend of the drug is prepared. The blend is generally accomplished using standard mixing or granulation processes known in the pharmaceutical industry. The blend is loaded into a tablet press specially designed for feeding tablets (or cores) and powders into a compression chamber wherein the powder or powders are compressed onto the tablet (or core). A die is generally selected to accommodate the particular tablet width allowing for the appropriate clearance parameters that will be discussed later. The active powder blend is metered using conventional tableting procedures with the mass of material determined by the height control of the press. The active powder blend in the die can optionally be tamped (i.e., precompressed). The coated core is placed in intimate contact with the powder blend. Optionally, a second layer is powder filled with the height adjustment appropriately above the tablet core height. A placebo may be preferred to minimize potency variability based on fill height variability (due to tablet core dimensional variability). In cases where this variability is not significant, or when the powder-added drug is the same as the controlled release drug, the top fill can be a drug. The combination is then pressed using dwell time and compression force to control the hardness (friability) of the added powder layers. The resultant tablet can optionally be overcoated with an additional coating to provide extra protection and/or a cosmetically appealing product. The particular shape of the core or tablet will be dependent upon the particular die used to manufacture the tablet and not limited by the present invention.

Each of the process steps described above and the materials used therein are discussed in more detail below.

### Pharmaceutical Core:

Any single- or multi-layered pharmaceutical tablet may be used and those skilled in the art will appreciate the applicability of the present invention to a wide variety of constructions. Two preferred examples of multi-layered cores include osmotic controlled release and enteric coated tablet cores. The CR core or tablet can be any pharmaceutical composition formulated for controlled or enteric release. The type of excipients used will depend upon the technology used, the desired delivery rate and the particular drug being administered. In general, the types of excipients, diluents and/or carriers that may be used are the same as those described above for the active powder coating. The pharmaceutically active ingredient can be any drug that one wants to incorporate into a delivery system where the release of the drug is controlled at a slower delivery rate in combination with another drug or the same drug at a faster delivery rate, or where the delivery is controlled by the position in the GI tract. Non-limiting examples of CR tablets that may be used include tablets of pseudoephedrine (U.S. Patent No. 6,171,618), GITS systems, such as Procardia^{™} XL and Glucotrol^{™} XL (available from Pfizer Inc., New York, NY), and other osmotic systems, such as Tegretol^{™} XR (available from Novartis Pharmaceuticals Corporation, East Hanover, NJ). A large variety of enteric coated products that are readily available commercially may be used in the inventive process. Alternatively, a placebo may be added instead of a drug.

Preparation of osmotic controlled release tablets is described in detail in Santus, G. and R. W. Baker, J. Control. Rel., **35**, 1-21 (1995) and references cited therein. Additional preparation information may be found in US Patent Nos. **4,857,331**; 4,327,725; 5,516,527; 5,612,059; 5,698,220; and 4,857,331; and U.S. Patent Application Serial No. 09/745095 entitled "Hydrogel-Driven Drug Dosage Form" filed by Appel, et al., on December 20, 2000.

In general, a tablet core is prepared by standard tableting processes either as a single layer, or as a bi- or tri-layer core. This core is then coated with a semi-permeable membrane, typically prepared from a combination of cellulose acetate and polyethyleneglycol (PEG). In some cases, a hole is made through the membrane using either mechanical drilling or laser drilling.

Preparation of enteric coated tablets involves film coating onto a compressed tablet core a material designed to dissolve at pH values above that typically found in the stomach. Descriptions of this process can be found in Lehmann, K., Manufac. Chem. & Aerosol News (1973); and Dreher, D., Pharma Internat, **1**(2), 3 (1975). Typical coating materials involve polymethacrylates such as Eudragits^{™} sold by Rohm GmbH Pharma Polymers (Darmstadt, Germany) and cellulose acetate hydrogen phthalate (CAP) sold by Eastman Chemical (Kingsport, TN).

### Compressible Coating

Tests were initially conducted by pressing a powder onto one side of a pharmaceutical core without the compressible coating in an attempt to adhere compressed IR materials to the walls of a conventional osmotic CR system. Compression forces were chosen to maximize the powder hardness without damaging the core. The primary excipient used in the CR core coating formulations was cellulose acetate. The IR tablet layer fell completely off the osmotic tablet coating in less than one hundred rotations of the friabilator. Numerous materials were tested as admixtures to the IR powder formulation. No pharmaceutically acceptable material in combination with the drug was found that would allow sufficient adhesion when compressed to the core to give acceptable friability without damaging the pharmaceutical core or compromising its dissolution performance. Moreover, this approach lacked generality given that the IR formulation must be tailored to adhesion and compression needs, thus limiting applicability when drugs themselves have tableting or stability issues. When a compressible material was coated onto the core, a significant improvement in adhesion of the IR powder to the coated core was observed. In particular, it was found that gum-based materials provided a low yield point coating while maintaining sufficient strength to effect adhesion. For example, Sentry^{™}Plus GB11 resin (available from Union Carbide, Danbury, CT) which softens slightly above room temperature provided a thin non-tacky compressible coating. GB11 is a polyvinyl acetate homopolymer having a softening point between about 48° and 50°C and a weight average molecular weight of about 10,000 (measured by gel permeation chromatography). The addition of a water-soluble plasticizer (e.g., PEG 3350) improved the water permeability of the coating and also improved the adhesion properties of the coating without adding unacceptable tack. The compressible coating of the present invention preferably contains a gum-based material(s) and a water-soluble plasticizer to provide a water permeable compressible coating. It will be understood by those skilled in the art that the synergistic effects of the combination of the gum-based materials with the water-soluble plasticizer play a role in defining the properties (tack, adhesion, water permeability, etc.) observed. Other materials may be used that meet the properties of the combined gum-based material and water-soluble plasticizer. In addition, an otherwise impermeable compressible coating can be made permeable by formation of pores or holes. Suitable techniques for making such holes include laser drilling, mechanical drilling, electroporation, etching, electron-beam treatment and the like. Other techniques include the application of a porous coating, such as a discontinuous film, a coating containing particulates, and a non-coalescing latex coating. Compressibility may also be imparted into a coating by making the coating porous such that the pores compress under tableting pressures. This property can therefore be a function of how a coating is made rather than the chemical composition of that coating. For example, a porous coating made by a precipitation process described in U.S. Patent No. 6,171,618. In some cases, it may be desirable to combine the low yield of a gum layer with the mechanical yield of a porous layer.

To evaluate the ability of a coating to compress under tableting pressures, a test was developed. The test is conducted using a model TA-HDi texture analyzer (Texture Technologies Corp., Scarsdale, NY) with a 5 kg load cell. A 3-mm diameter stainless steel probe was used with a 1 g trigger, a compression speed of 0.50 mm/sec to a distance of 0.10 mm. The force in grams is measured at the 0.05 mm depth. Pharmaceutical cores coated with a compressible coating having a force of less than about 500 g can be successfully compressed with a powder to form a compressed tablet. For example, a commercially available Procardia^{™} XL (90 mg GITS) tablet had a value of 600 g; however, the compressibility of the tablet was reduced to 490 g by applying a 2% 7:3 GB11:PEG 3350 coating onto the surface of the tablet. A commercially available Tegretol^{™} XR (400 mg tablet) had a value of 800 g. The compressibility of the Tegretol^{™} tablet was reduced to 130 g by applying a 0.1 mm coating of 7:3 GB11:PEG 3350. The application of a porous coating made from cellulose acetate using a procedure described in U.S. Patent No. 6,171,618 provided a compressibility value of 400 g.

Another desirable feature of the compressible coated cores is its ability not to stick to each other during handling. One can test this feature by allowing tablets to sit in a bottle for at least 24 hours and then visually examining the tablets to see if any tablets stuck to each other such that more than a minimal force is needed to separate the tablets.

The following three properties assist in identifying suitable materials and coating thicknesses for use as a compressible coating: (1) the yield pressure which is an indication of the ability of the material to deform during powder compression is preferably less than about 350 MPa, more preferably less than about 100 MPa and most preferably less than about 50 MPa; alternatively, one can use the functional test described above where the coating will provide a force of less than about 600 g, more preferably less than about 500 g and most preferably less than about 450 g; (2) the material preferably has sufficient strength and sufficiently low tack such that it can be handled in the subsequent steps (e.g., the coating preferably does not fracture or chip under handling and storage conditions and the tablets do not stick to each other or to the containers used for storage); and (3) the coating is preferably sufficiently permeable and/or disintegratable to allow the desired controlled release behavior of the core in the use environment beyond levels that make the performance of the core unacceptable. It is preferred that the release rate after coating be between about 0.25 and about 3 times the release rate before coating; more preferably between about 0.75 and about 1.5, and most preferably between about 0.8 and about 1.3. Suitable coating materials include materials that are solvent or water soluble, liquid dispersions, latexes, or combinations thereof. These coatings can themselves dissolve or decompose in the use environment. They may also release from the core coatings in the use environment. Suitable compressible (i.e., low yield point) materials include materials such as polyvinyl acetates having a weight average molecular weight from about 2,000 to about 20,000 and a softening point between about 40° to about 70°C (e.g., Sentry^{™} GB11 - softening point between 48° and 50°C available from Union Carbide; and Vinnapas^{™} B1.5-MW 10,000-15,000 as measured by size exclusion chromatography and softening range from about 60° to about 64°C available from Wacker-Chemie GmbH, München, Germany). Other suitable materials can include such low yield materials as ethylene vinyl acetate copolymers; poly(ethylene oxides); poly(propylene oxides); copolymers of poly(ethylene oxides) and poly(propylene oxides); and other gum based materials such as those detailed in Section 172.615 in the US Code of Federal Regulations, Title 21. Suitable plasticizers (when needed) include polyethylene glycols (e.g., PEG 3350); diethyl phthalate and other phthalate esters; glycerol and glycerol esters; propylene glycol and esters thereof; stearic acid and salts thereof; and triacetin, and combinations thereof. In some cases, combinations of water soluble and insoluble plasticizers can be used.

A typical compression layer would contain from about 50% to about 100% low yield point polymeric material. For example, when GB11 is used as the polymeric material, then the compression layer would typically contain from about 50% to about 99% GB11 preferably about 60% to about 95%, more preferably about 60% to about 80%. The plasticizer, if necessary, is generally present in an amount from about 1 % to about 50%, preferably about 5% to about 40%, more preferably about 10% to about 40%. The exact ratio will depend upon the desired tack, water-permeability and compressibility of the coating for the particular drugs both in the powder formulation and in the core. Those skilled in the art will know how to adjust the ratio to achieve the desired properties for the particular materials used in the formulation. The low yield point material and the plasticizer (if needed) are solubilized or dispersed in a solvent or mixture of solvents and then coated onto the pharmaceutical core. The coating solution may be applied using any liquid coating method generally know to those skilled in the art. Typical means of coating include spray-coating in a pan coater and fluid bed coating. Suitable solvents include water, ketones (e.g., acetone), alcohols, esters, amides, ethers, and mixtures thereof. The solvent is removed with forced-air heat to yield a CR tablet having a water-permeable compressible coating with a thickness from about 5 µm to about 500 µm, preferably from about 5 µm to about 200 µm, more preferably from about 10 µm to about 100µm, even more preferably from about 20 µm to about 70 µm, most preferably from about 30 µm to about 50 µm. Other additives may be incorporated into the compressible coating such as stabilizers (e.g., antioxidants), flavoring agents, lubricants (e.g., talc), colorants and other materials well known to those skilled in the art. The coating may be a continuous coating surrounding the core or a partial coating. For example, the tablet could be dipped into the coating solution such that only a part of the tablet is coated with the coating solution.

An alternative method for imparting permeability to the coating involves forming a porous coating. The techniques for forming porous coatings include coating from solvent mixtures such that the material is more soluble in the lower boiling solvent such that a non-continuous film forms (precipitation) during coating; coating a dispersion or latex under conditions which do not allow for coalescence either by remaining below the material's glass transition temperature or by adding materials which prevent the coalescence; adding materials to the layer which leach out in the use environment such that pores remain in the coating. Suitable materials include water soluble low molecular weight materials such as salts and sugars. In addition, an otherwise impermeable compressible coating can be made permeable by formation of pores or holes as described earlier.

In addition, it was found that the compressible (polymeric) coating can function as a delayed or controlled release coating. Therefore, another aspect of the present invention is the use of the compressible coating described above as a delayed or controlled release coating. Consequently, a tablet having a compressible coating that functions as a delayed or controlled release with or without the addition of the active powder is also within the scope of the present invention. The drug release profile may be adjusted by varying the ratio of the gum-based material and the water soluble plasticizer, and the thickness of the coating. The release profile may also be adjusted by varying the type and/or molecular weight of the gum-based material, as well as the type and water solubility of the plasticizer, as exemplified with the non-gum based membranes described in US Patent No. 6,171,618. Those skilled in the art will know how to adjust the formulation to achieve the desired release profile. Additional coatings may be applied over the compressible coating to impart additional desired properties to the tablet (e.g., to provide protection, to add flavor, to add color, to provide a printable surface, to aid swallowing, etc.).

It will be appreciated by those skilled in the art that water permeability, and therefore, the rate of drug release from a coated core can depend on the amount of water soluble plasticizer and the thickness of a coating when using a water insoluble film coating on a pharmaceutical core. It was found that even for very thin coatings, a low molecular weight poly(vinylacetate) (GB11) requires some plasticization with PEG to allow any drug to be released. By adjusting the ratio of GB11 to PEG and the thickness, a thin (2 weight percent) coating can slow drug delivery to allow for delivery over extended periods (12-18 hours) or be made to have virtually no impact on the drug release profile.

The inventive compressible coating provides several advantages over conventional cellulose-based coatings. For example, the inventive compressible coating can collapse after the bulk of the drug is delivered into the GI tract. An additional advantage is the ability to imprint (emboss) a form of identification onto the final tablet surface. This identification can be used as an anti-counterfeit method or for brand identification. Although it is sometimes possible to put such identification into the original, uncoated tablet core, such surfaces can often result in discontinuous coatings which may split in the GI tract. As such, there is a distinct advantage to being able to put a compressible, functional coating on a tablet.

### Drug Powder Formulation

One of the key advantages of the present invention is the flexibility in terms of the drug and excipient used in the drug powder. For example, the drug powder could be an IR formulation thus allowing immediate release of a first drug followed by the osmotic CR or enteric delivery of a second drug in the tablet core. Alternatively, the active powder could be a CR matrix which would allow for a controlled release with one rate followed by an osmotic CR delivery with a different rate. The drugs in the powder and core can be the same or different.

The drug powder formulation may include one or more pharmaceutically acceptable excipients, carriers or diluents. Excipients are generally selected to provide good compression profiles under direct compression. The particular carrier, diluent or excipient used will also depend upon the desired delivery profile. In general, a tablet formulation includes materials such as diluents, binders, lubricants, disintegrants and mixtures thereof. Suitable diluents include various types of starch, lactose, mannitol, kaolin, calcium phosphate or sulfate, inorganic salts (e.g., sodium chloride), powdered sugar, and powdered cellulose derivatives. A preferred diluent for powders containing cetirizine is microcrystalline cellulose (e.g., Avicel® PH102 available from FMC Pharmaceutical, Philadelphia, PA).

If desired, suitable tablet binders include substances such as celluloses (e.g., cellulose, methylcellulose, ethylcellulose, and hydroxymethylcellulose), polypropylpyrrolidone, polyvinylprrolidone, gelatin, gum arabic, polyethylene glycol, starch, sugars (e.g., lactose, sucrose, fructose, and glucose), natural and synthetic gums (e.g., acacia, alginates, and gum arabic) and waxes.

A lubricant is typically used in a tablet formulation to prevent the tablet and punches from sticking in the die. Suitable lubricants include slippery solids such as talc, magnesium and calcium stearate, stearic acid, light anhydrous silicic acid, and hydrogenated vegetable oils. A preferred lubricant is magnesium stearate.

Tablet disintegrators which swell when wetted are added to the composition to break up the immediate release portion of the dosage form and release the compound. Suitable disintegrants include starches (e.g., corn or potato starches and hydroxypropylstarch), clays, celluloses (e.g., cellulose, wood cellulose, methyl- or ethyl-cellulose, low substituted hydroxypropylcellulose, and carboxymethylcellulose), agar, algins (e.g., alginic acid), powdered natural sponge, cation-exchange resins, citrus pulp, bentonite, sodium bicarbonate, calcium phosphate, calcium citrate, sodium lauryl sulfate, and gums (e.g., guar gum). Preferred disintegrators are sodium starch glycolate (e.g., Explotab® available from Mendell, Patterson, NY) and croscarmellose sodium (Ac-Di-Sol® available from FMC Pharmaceuticals). Most preferred is croscarmellose sodium.

Other useful additives include materials such as agents for retarding dissolution (e.g., paraffin), resorption accelerators (e.g., quaternary ammonium compounds), surface active agents (e.g., cetyl alcohol, glycerol monostearate, and sodium lauryl sulfate), adsorptive carriers (e.g., kaolin and bentonite), preservatives, sweeteners, coloring agents, flavoring agents (e.g., citric acid, menthol, glycine or orange powder), stabilizers (e.g., citric acid, sodium citrate or acetic acid), dispersing agents (e.g., hydroxypropylmethylcellulose), and mixtures thereof.

The amount of active ingredient applied via the active powder is controlled by using the height control in the die as is commonly done for solid tableting. The exact amount to be applied will depend on the powder density and the desired unit dose to be administered *via* the faster delivery rate.

Powder compositions for the immediate release compression formulation can be produced by any of the means that are well known in the art to provide the desired uniformity and distribution of the drug. These include direct compression by simple blending of components and granulations. The granulation can be a dry granulation (e.g., roller compaction), or a wet granulation (e.g., low or high shear granulation and fluidized bed).

### Compressing the Active Powder onto the Pharmaceutical Core

In one embodiment of the present invention, the compressed powder is added to the pharmaceutical core such that the overall tablet size is minimized. Preferably, this approach is used when the core diameter dimensional variability is low. For this approach, a punch and die are chosen such that their widths are as narrow as possible while still allowing adequate tolerance for the tablet to be fed into the die. Therefore, the size of the die is generally slightly larger than the diameter of the coated tablet. Since the tablet is often forced into the die by an insertion punch (e.g., Pharmapress^{™} PH 800 rotary press available from Korsch^{™}, Berlin, Germany), the tolerance needed is about **0.102 mm (0.004 inch)** in diameter beyond any tolerances for core variability. This will minimize any deformation of the tablet or cracking of the tablet wall. The dwell times and compression forces are optimized for the given formulation to achieve minimal friability of the compressed powder without detrimental effects on the drug delivery profiles of the CR dosage form. Those skilled in the art will know how to adjust the settings for their particular equipment to achieve optimum results. The coated tablet is placed in the die with as much accuracy as possible. For example, the Pharmapress^{™} PH 800 rotary press grabs tablets with a vacuum transport and places them to a precision of **0.102 mm (0.004 inch)** in all directions.

In another embodiment of the present invention, a second powder coating is applied after the insertion of the coated tablet. The second powder coating is then compressed onto the top of the tablet at the same time as the first powder coating is compressed onto the bottom of the tablet. This allows even more flexibility in terms of multiple drugs or release rates. For example, the second powder coating can contain: (i) a third drug with a release rate different from the first powder coating or the CR core; (ii) the same drug as the first powder coating having the same or a different release rate; (iii) the same drug as the CR core having the same or different release rate; or (iv) a placebo where the coating provides cushioning during the compression step and/or additional protection against sticking to the die. Preferably, a placebo is used as the second powder since this would reduce the effects of any core dimensional variability on the potency of the actives in the final dosage form. Preferably, sufficient clearance of the core from the die walls is available to allow powder to flow from the top around the sides of the tablet. The lower limits of the clearance will depend upon the particle size of the powder. The upper limit is generally no more than about **0.635 cm (0.25 inches)**. Preferably, the die dimension is more than about **0.0076 mm (0.0003 inch)**, more preferably about **0.305 mm (0.012 inch)** to about **0.889 mm (0.035 inch)** greater in diameter than the diameter of the cores. This also suggests that the placebo formulation is preferably chosen to minimize the particle size dimensions while allowing adequate flow.

In general, as the thickness of the powder layer increases with respect to the size of the pharmaceutical core, the need for a compressible coating decreases. For example, U. S. Patent No. 6,136,345 describes a pharmaceutical core with a non-compressible coating to which a powder is compressed on the outside. However, in that system, the weight of the compressed powder in the two examples was approximately 125% and 160% of the core weight (i.e., a weight ratio of compressed layer to core of 1.25:1 and 1.6:1, respectively). When the compressed powder coating is less than the weight of the pharmaceutical core, it very difficult if not impossible to successfully compress a powder onto the core without adverse results (e.g., delamination of the powder from the core or friability test failure). However, when the compressible coating of the present invention is applied to the core prior to compressing the powder onto the tablet, the powder coating was successfully compressed onto the core at a weight as low as 33 wgt.% of the core weight with no delamination of the powder from the pharmaceutical core or loss of material from the tablet upon friability testing. Consequently, one aspect of this invention is the ability to apply lower amounts of a compressed layer onto a pharmaceutical core (or tablet). Preferably, the amount of compressed layer is from about 5 wgt% to less than about 125 wgt% of the core (i.e., weight ratio of about 1:20 to less than about 1.25:1), more preferably from about 10 wgt% to about 100 wgt% (i.e., ratio of about 1:10 to about 1:1), and most preferably from about 20 wgt% to about 90 wgt% (i.e., weight ratio of about 1:5 to about 9:10).

After compression, the tablets are ejected from the die and packaged. Alternatively the tablets may be overcoated with an additional film coating. The additional coating may serve to mask the taste of the drug, provide for easier swallowing, impart chemical or physical stability, provide an enteric coating or allow for identification (e.g., by providing a specific color, or printed logo or trademark). The additional film coating can be applied by any conventional film coating process well known to those skilled in the art (e.g., spray coating in a pan or fluidized bed coating).

### Packaging

The pharmaceutical tablets may be packaged in a variety of ways. Generally, an article for distribution includes a container which contains the pharmaceutical tablets. Suitable containers are well-known to those skilled in the art and include materials such as bottles (plastic and glass), plastic bags, foil packs, and the like. The container may also include a tamper-proof assemblage to prevent indiscreet access to the contents of the package. In addition, the container has deposited thereon a label that describes the contents of the container and any appropriate warnings.

The following Examples illustrate the methods of the present invention and the pharmaceutical tablets produced therefrom. To illustrate the general concepts of the present invention, specific pharmaceutically active ingredients are used. However, those skilled in the art will appreciate that the particular drug used is not limiting to the scope of the invention and should not be so construed.

### EXAMPLES

The following materials used in the Examples may be obtained from the corresponding sources listed below:

| | |
|---|---|
| pseudoephedrine HCl | Knoll Fine Chemicals (New York, NY) |
| cetirizine HCl | Pfizer Inc. (New York, NY) |
| pseudoephedrine HCl Tablets U.S. Patent No. 6,171,618 | prepared according to |
| GB11 polyvinyl acetate resin | Union Carbide (Danbury, CT) |
| PEG 3350 (polyethylene glycol) | Union Carbide (Danbury, CT) |
| Avicel^{™} PH102 | FMC Pharmaceutical (Philadelphia, PA) |
| (microcrystalline cellulose) | |
| Klucel^{™} EXF | Hercules (Aqualon^{™}) (Wilmington, DE) |
| (hydroxypropylcellulose) | |
| Lactose Fast Flo 316 | Foremost Corp. (Baraboo, WI) |
| Cab-O-Sil^{™} (silicon dioxide) | Cabot Corp. (Tuscola, IL) |
| Magnesium stearate | Mallinckrodt (St. Louis, MO) |

Tablet cores were all prepared using a Manesty^{™} F-Press (single-punch tablet machine available from Manesty Corporation, Liverpool, UK). Use of such tablet presses is described in Pharmaceutical Dosage Forms: Tablets, Volume 2 (H. A. Leberman, L. Lachman, J. B. Schwartz, Eds.), Marcel Dekker, Inc. New York (1990).

### Example 1

Tablets were prepared by combining an immediate release formulation of cetirizine with a controlled release formulation of pseudoephedrine. Pseudoephedrine osmotic CR tablets (240 mg, **1.11 cm (7/16")** SRC cores with 20 wgt% asymmetric membrane (*AM* coating) were coated with the following formulation to a dry weight of approximately 2.5 wgt%.

| | |
|---|---|
| GB11 | 1.8% |
| PEG 3350 | 0.75% |
| Water | 5.4% |
| Acetone | 92.0% |

The coating solution was prepared by combining all the above ingredients and stirring till the solution became clear (about 2 hours). The coating solution was applied to the core tablets in a pan coater (HCT30; Vector Corp.). The coating was carried out with an inlet temperature of 28°C, an outlet temperature of 23°C, an airflow of **0.02 m**^{**3**}**/s(41 CFM),** and with liquid pumped to the spray nozzle at a rate of 7 g/min. After the target weight was achieved, the tablets were removed and placed on polyethylene lined trays where they were oven dried for 18 hrs at 40°C

Using an F-press (Manesty Corp.) with **1.219 cm (0.480")** SRC die and punch, 80 mg of the following powder was added to the die:

| | |
|---|---|
| Avicel PH102: | 74.6% |
| cetirizine HCl: | 24.9% |
| magnesium stearate | 0.5% |

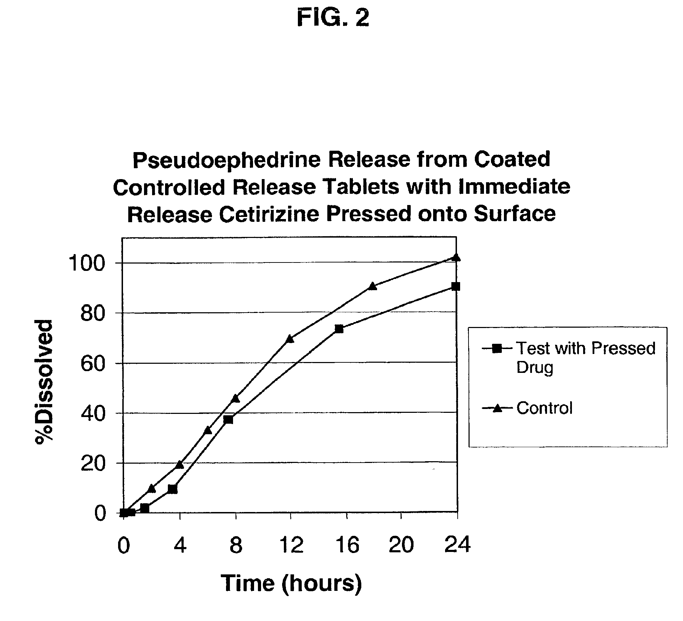

The powder blend was prepared by bottle blending the Avicel and cetirizine for 15 minutes, then adding the magnesium stearate and blending for an additional 5 minutes. A coated pseudoephedrine tablet from above was added to the die then the combination was pressed effectively adhering the cetirizine powder to the tablet. There were no obvious cracks in the coatings. Friability testing indicated no weight loss after 200 revolutions and no visible signs of damage to the IR layer. Dissolution experiments were conducted using 500 mL of deionized water at 37°C, 50-rpm paddle speed. Analysis was performed by UV absorption (λ = 232 nm for cetirizine and 258 nm for pseudoephedrine). The cetirizine is all dissolved within one hour. The dissolution curve of the pseudoephedrine is shown in Figure 2. As can be seen, there is a delay in the initial drug release followed by a period of several hours where the rate of drug release appears similar to the control. The time to deliver 50% of the drug was about 9 hours for the control and about 10 hours for the test tablets. Both test and control deliver comparable amounts of drug after 24 hours.

Example 2 illustrates the use of a second powder coating applied to the opposite side of the tablet.

### Example 2

The following formulations were prepared as described below.

### Pseudoephedrine tablet blend:

| Part A: | |
|---|---|
| pseudoephedrine HCl (crystals) | 75.4% |
| Avicel^{™} PH101 (MCC) | 21.2% |
| Klucel^{™} EXF (HPC) | 3.4% |
| Water | (8.%) |

A 2 kg batch was prepared by first dry mixing the above powders in an SP-1 high shear granulator (Niro Corp., Columbia, MD) for 5 minutes. The water (160 g) was added at 60 g/min, then the sample was mixed for an additional 30 sec. to give a total mix time of 8 min. The granulation was then spread on a polyethylene lined tray and dried 16 h at 50°C. The granulation was then passed through an M5A mill (Fitzpatrick Corp.) using a **0.084 cm (0.033")** conidur rasp at 300 rpm. A final pseudoephedrine tablet blend (containing 44.9% pseudoephedrine) was prepared by combining the components below without the magnesium stearate at the designated percentages and blending in a V-blender for 15 minutes followed by an additional 5-minutes after addition of the magnesium stearate.

| | |
|---|---|
| pseudoephedrine granulation- Part A | 59.5% |
| Avicel^{™} PH200 (MCC) | 40.0% |
| magnesium stearate | 0.5% |

The blended composition was then formed into tablets using an F-press (Vector Corp.).

### Asymmetric Membrane (AM) Osmotic Coating:

Approximately 1 kg of tablet cores were placed in an HCT-30EP Hicoater (Niro Corp.). The inlet temperature was set to 48°C to maintain a 27°C outlet temperature. A coating solution prepared by dissolving PEG 3350 (90 g) in water (1035 g) then adding cellulose acetate (360 g) and acetone (3015 g) slowly with vigorous stirring. This coating solution was applied in the coater at a spray rate of 20 g/min with a pan speed of 15 rpm, a gun at **7.6 cm (3 inches)** above the tablet bed, an atomizing air pressure of **0.14 MPa (20 psi)**. The coating was stopped when a 30 weight percent increase was achieved. The tablets were spread on a polyethylene lined tray and dried in an oven at 50°C for 16 hours.

### Water-permeable compressible coating:

A coating solution was prepared by dissolving 15 g of PEG 3350 in 25 g of water, then adding 35 g of GB11 and 425 g of acetone with vigorous stirring till the mixture dissolved. Approximately 1 kg of the coated tablets from above were placed in an HCT-30EP Hicoater. The inlet temperature was set to 35-38°C to maintain an outlet temperature of 27-28°C. The coating solution was applied at 7 g/min with a pan speed of 15 rpm, a gun distance of **7.6 cm (3 inches),** atomizing air pressure of **0.14 MPa (20 psi)**. Coating continued until 315 g of solution were added to give a 2-2.5 weight percent coating. Tablets were then placed on a polyethylene lined tray and dried at 40°C for 16 hours.

### Cetirizine powder blend:

| | |
|---|---|
| cetirizine HCl | 20.0% |
| lactose Fast Flo 316 | 50.5% |
| Avicel^{™} PH102 (microcrystalline cellulose) | 28.1% |
| Cab-O-Sil^{™} (silicon dioxide) | 0.4% |
| magnesium stearate | 1.0% |

All the powders except the magnesium stearate were combined and mixed for 20 minutes in a V-blender. The magnesium stearate was then added and the mixture was blended in the V-blender for an additional 5 minutes. The powder blend was stored in a polyethylene bag.

### Placebo powder blend:

| | |
|---|---|
| lactose Fast Flo 316 | 63.4% |
| Avicel^{™} PH102 (microcrystalline cellulose) | 35.1% |
| Cab-O-Sil^{™} (silicon dioxide) | 0.5% |
| magnesium stearate | 1.0% |

All the powders except the magnesium stearate were combined and mixed for 20 minutes in a V-blender. The magnesium stearate was then added and the mixture was blended in the V-blender for an additional 5 minutes. The powder blend was stored in a polyethylene bag.

### Compression of Powders onto Cores

Using a Korsch 800 core coater, the cetirizine powder blend was fed into the lower die (approximately 70 mg). Tablets of pseudoephedrine coated with the osmotic and compressible coatings above were then placed onto the powder blend in the die. In some tests, placebo blend was then fed on top of the tablet with the lower punch adjusted to fill about 75 mg. The powders were then compressed to provide the final combination tablets. Table 1 below compares the mean weight, tablet diameter, tablet thickness, coating thickness and height of the tablet at various stages listed below:
Sample 2-1: Pseudoephedrine tablets, no coatings
Sample 2-2: Pseudoephedrine tablets, coated with a 30.0 wgt.% AM coating
Sample 2-3: Sample 2-2 coated with a 2.3 wgt.% water-permeable compressible coating.
Sample 2-4: Sample 2-3 compressed with only the cetirizine powder (approximately 70 mg).
Sample 2-5: Sample 2-3 compressed with both the cetirizine powder and placebo powder (approximately 145 mg total for the powders; compression force = 24 kilonewtons, kN).
Sample 2-6: Sample 2-3 compressed with both the cetirizine powder and placebo powder (approximately 145 mg total for the powders; compression force = 11 kN).

**TABLE 1**

| **Sample No.** | **Mean Wt. (mg)** | **Tablet Diameter (mm)** | **Tablet Thickness (mm)** | **Coating Thickness (osmotic/ compressible)** (µm) | **IR Powder Thickness (µm)** |
|---|---|---|---|---|---|
| 2-1 | 180 | 7.9 (5/16") | 4.1 | --- | |
| 2-2 | 236 | 9.0 | 5.0 | 590 | --- |
| 2-3 | 243 | 9.1 | 5.1 | 590/42 | --- |
| 2-4 | 243 | 9.5 | 4.3 | --- | IR powder fell off and core cracked |
| 2-5 | 390 | 10 | 4.5 | 280/29 | 500 |
| 2-6 | 350 | 10 | 4.6 | 280/42 | 600 |

Two different compression forces were used, 24kN and 11 kN, to encase the coated pseudoephedrine cores in cetirizine and placebo blend using 10-mm tooling. An attempt to apply cetirizine only to one side of the cores (sample 2-4) failed and the cores cracked from the force. It is believed that this was due to the mismatch of curvatures of the tablet cores and the punch. This mismatch, where the tablet curvature is greater than that for the punch, required greater pressures to provide adequate compression at the sides. The unevenness of the pressure resulted in coating cracks. Though this is an extreme test where the tablet cores and die and punch are vastly different, the addition of the placebo top fill resulted in acceptable performance. The effect of the two different compression forces on the tablets was not significantly different from each other. They both reduced the tablet thickness approximately 20% from the in-going core size. The osmotic coating thickness was reduced approximately 50%, indicative of compression of that coating. There did not appear to be an effect on the gum layer.

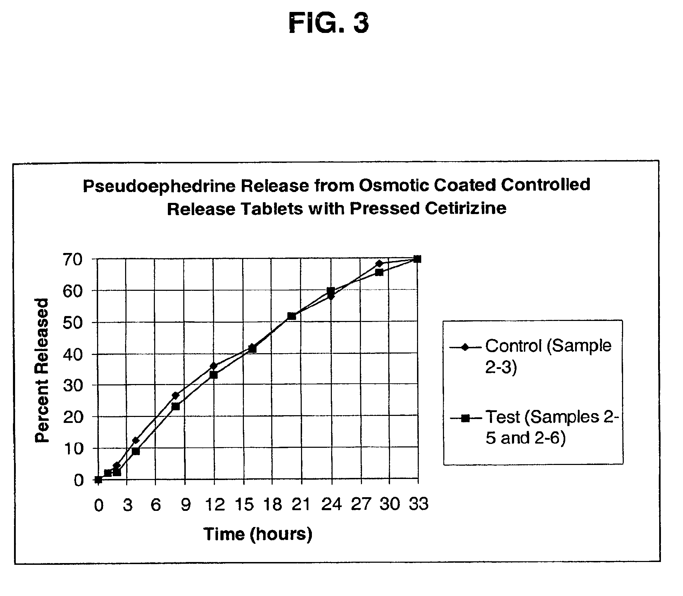

An average of nine tablets from both Samples 2-5 and 2-6 had a potency for cetirizine of 13.3 ± 0.7 mg/tablet (RSD = 5.1%). The potency was determined by UV-spectroscopy. The dissolution properties were determined in 500-mL of distilled water at 37°C and 50 rpm. The percent drug released versus time for an average of 2 control samples (Sample 2-3) and an average of 6 test samples (Samples 2-5 and 2-6) is shown in Figure 3.

## Claims

1. A pharmaceutical tablet comprising a pharmaceutical osmotic controlled release core containing a placebo or a first drug and having a top side, a bottom side and edges, a compressible coating deposited on said core, and a second drug compressed onto said compressible coating adjacent to said bottom side of said core to form a first compressed layer.

2. The pharmaceutical tablet of Claim 1 further comprising a placebo or a third drug compressed onto said compressible coating adjacent to said top side to form a second compressed layer.

3. The pharmaceutical tablet of Claim 2 wherein the weight ratio of the combined said first compressed layer and said second compressed layer to said pharmaceutical core is from 1:20 to less than 1.25:1.

4. The pharmaceutical tablet of Claim 1, 2 or 3 wherein said second drug is the same as said first drug.

5. The pharmaceutical tablet of Claim 1, 2 or 3 wherein said second drug is different from said first drug.

6. The pharmaceutical tablet of any one of the preceding claims wherein said second drug has a controlled release drug delivery profile.

7. The pharmaceutical tablet of Claim 1, 2, 3, 4, or 5 wherein said second drug has an immediate release rate.

8. A pharmaceutical tablet of any of the preceding claims wherein said compressible coating functions as a controlled release coating.

9. The pharmaceutical tablet of any one of the preceding claims wherein said compressible coating comprises a gum-based resin.

10. The pharmaceutical tablet of Claim 9 wherein said gum-based resin is a polyvinylacetate resin having a weight average molecular weight from 2,000 to 20,000.

11. A method for manufacturing a pharmaceutical tablet comprising the steps of:
(i) providing a pharmaceutical osmotic controlled release core containing a placebo or a first drug and having deposited thereon a compressible coating;
(ii) placing a first powder comprising a second drug into a die press;
(iii) placing said pharmaceutical core from step (i) in intimate contact with said first powder in said die press;
(iv) compressing said first powder onto said compressible coating on said core to form a compressed tablet; and
(v) optionally, coating said compressed tablet from step (iv) with an outer coating.

12. A method for manufacturing a pharmaceutical tablet comprising the steps of:
(i) providing a pharmaceutical osmotic controlled release core containing a placebo or a first drug and having deposited thereon a compressible coating;
(ii) placing a first powder into a die press;
(iii) placing said pharmaceutical core from step (i) in intimate contact with said first powder in said die press;
(iv) placing a second powder in intimate contact with said pharmaceutical core on the opposite side from said first powder in said die press;
(v) compressing both said first powder and said second powder onto said compressible coating on said core to form a compressed tablet; and
(vi) optionally, coating said compressed tablet from step (v) with an outer coating
wherein either said first powder or said second powder comprises a second drug.

## Patentansprüche

1. Pharmazeutische Tablette, umfassend einen pharmazeutischen osmotisch kontrollierten Freisetzungskern, enthaltend ein Placebo oder ein erstes Arzneimittel mit einer Oberseite, einer Unterseite und Ecken, einem auf dem Kern abgeschiedenen komprimierbaren Überzug, und ein zweites Arzneimittel, das auf dem komprimierbaren Überzug, der Unterseite des Kerns benachbart, zur Bildung einer ersten zusammengepressten Schicht zusammengepresst ist.

2. Pharmazeutische Tablette nach Anspruch 1, weiterhin umfassend ein Placebo oder ein drittes Arzneimittel, das auf dem komprimierbaren Überzug, benachbart zu der Oberseite, zur Bildung einer zweiten zusammengepressten Schicht zusammengepresst ist.

3. Pharmazeutische Tablette nach Anspruch 2, worin das Gewichtsverhältnis der vereinigten ersten zusammengepressten Schicht und der zweiten zusammengepressten Schicht zu dem pharmazeutischen Kern im Bereich von 1:20 bis weniger als 1,25:1 liegt.

4. Pharmazeutische Tablette nach Anspruch 1, 2 oder 3, worin das zweite Arzneimittel das gleiche wie das erste Arzneimittel ist.

5. Pharmazeutische Tablette nach Anspruch 1, 2 oder 3, worin das zweite Arzneimittel von dem ersten Arzneimittel verschieden ist.

6. Pharmazeutische Tablette nach einem der vorhergehenden Ansprüche, worin das zweite Arzneimittel ein kontrolliertes Arzneimittel-Freisetzungsprofil aufweist.

7. Pharmazeutische Tablette nach Anspruch 1, 2, 3, 4 oder 5, worin das zweite Arzneimittel eine sofortige Freisetzungsrate aufweist.

8. Pharmazeutische Tablette nach einem der vorhergehenden Ansprüche, worin der komprimierbare Überzug als Überzug zur kontrollierten Freisetzung fungiert.

9. Pharmazeutische Tablette nach einem der vorhergehenden Ansprüche, worin der komprimierbare Überzug ein Harz auf Gummi- bzw. Kautschukbasis umfasst.

10. Pharmazeutische Tablette nach Anspruch 9, worin das Harz auf Gummi- bzw. Kautschukbasis ein Polyvinylacetatharz mit einem durchschnittlichen Molekulargewicht von 2.000 bis 20.000 ist.

11. Verfahren zur Herstellung einer pharmazeutischen Tablette, umfassend die Stufen:
(i) der Bereitstellung eines pharmazeutischen Kerns zur osmotischen kontrollierten Freisetzung, enthaltend ein Placebo oder ein erstes Arzneimittel und darauf aufgebracht einen komprimierbaren Überzug;
(ii) des Einbringens eines ersten Pulvers, umfassend ein zweites Arzneimittel in ein Presswerkzeug;
(iii) des in innigen Kontaktbringens des pharmazeutischen Kerns aus Stufe (i) mit dem ersten Pulver in der Pressform;
(iv) des Verpressens des ersten Pulvers auf den komprimierbaren Überzug auf dem Kern zur Herstellung einer verpressten Tablette; und
(v) gegebenenfalls des Überziehens der verpressten Tablette aus Stufe (iv) mit einem äußeren Überzug.

12. Verfahren zur Herstellung einer pharmazeutischen Tablette, umfassend die Stufen:
(i) der Bereitstellung eines pharmazeutischen Kerns zur osmotischen kontrollierten Freisetzung, enthaltend ein Placebo oder ein erstes Arzneimittel und darauf aufgebracht einen komprimierbaren Überzug;
(ii) des Einbringens eines ersten Pulvers in eine Pressform;
(iii) des in innigen Kontaktbringens des pharmazeutischen Kerns aus Stufe (i) mit dem ersten Pulver in der Pressform;
(iv) des in innigen Kontaktbringens eines zweiten Pulvers mit dem pharmazeutischen Kern auf der entgegengesetzten Seite von dem ersten Pulver in die Pressform;
(v) des Verpressens sowohl des ersten Pulvers und des zweiten Pulvers auf den komprimierbaren Überzug auf dem Kern zur Herstellung einer verpressten Tablette; und
(vi) gegebenenfalls des Überziehens der verpressten Tablette aus Stufe (v) mit einem äußeren Überzug,
worin entweder das erste Pulver oder das zweite Pulver ein zweites Arzneimittel umfasst.

## Revendications

1. Comprimé pharmaceutique comprenant un noyau pharmaceutique, à libération commandée osmotique, contenant un placebo ou un premier médicament et ayant une face supérieure, une face inférieure et des bords, un enrobage compressible déposé sur ledit noyau, et un second médicament compressé sur ledit enrobage compressible au voisinage de ladite face inférieure dudit noyau pour former une première couche compressée.

2. Comprimé pharmaceutique selon la revendication 1, comprenant, en outre, un placebo ou un troisième médicament compressé sur ledit enrobage compressible au voisinage de ladite face supérieure pour former une seconde couche compressée.

3. Comprimé pharmaceutique selon la revendication 2, dans lequel le rapport pondéral entre ladite première couche compressée et ladite seconde couche compressée, combinées, et ledit noyau pharmaceutique va de 1:20 à moins de 1,25:1.

4. Comprimé pharmaceutique selon la revendication 1, 2 ou 3, dans lequel ledit second médicament est identique audit premier médicament.

5. Comprimé pharmaceutique selon la revendication 1, 2 ou 3, dans lequel ledit second médicament diffère dudit premier médicament.

6. Comprimé pharmaceutique selon l'une quelconque des revendications précédentes, dans lequel ledit second médicament a un profil de distribution de médicament à libération commandée.

7. Comprimé pharmaceutique selon la revendication 1, 2, 3, 4 ou 5, dans lequel ledit second médicament est à libération immédiate.

8. Comprimé pharmaceutique selon l'une quelconque des revendications précédentes, dans lequel ledit enrobage compressible agit comme enrobage à libération commandée.

9. Comprimé pharmaceutique selon l'une quelconque des revendications précédentes, dans lequel ledit enrobage compressible comprend une résine à base de gomme.

10. Comprimé pharmaceutique selon la revendication 9, dans lequel ladite résine à base de gomme est une résine de poly(acétate de vinyle) ayant un poids moléculaire moyen en poids allant de 2000 à 20000.

11. Procédé de fabrication d'un comprimé pharmaceutique comprenant les étapes de :
(i) fourniture d'un noyau pharmaceutique, à libération commandée osmotique, contenant un placebo ou un premier médicament et sur lequel est déposé un enrobage compressible ;
(ii) mise en place d'une première poudre comprenant un second médicament dans une presse à matricer ;
(iii)mise en place dudit noyau pharmaceutique issu de l'étape (i) en contact intime avec ladite première poudre dans ladite presse à matricer ;
(iv) compression de ladite première poudre sur ledit enrobage compressible, sur ledit noyau, pour former un comprimé compressé ; et
(v) facultativement, enrobage dudit comprimé compressé issu de l'étape (iv) avec un enrobage extérieur.

12. Procédé de fabrication d'un comprimé pharmaceutique comprenant les étapes de :
(i) fourniture d'un noyau pharmaceutique, à libération commandée osmotique, contenant un placebo ou un premier médicament et sur lequel est déposé un enrobage compressible ;
(ii) mise en place d'une première poudre dans une presse à matricer ;
(iii)mise en place dudit noyau pharmaceutique issu de l'étape (i) en contact intime avec ladite première poudre dans ladite presse à matricer ;
(iv) mise en place d'une seconde poudre en contact intime avec ledit noyau pharmaceutique sur la face opposée à celle de ladite première poudre dans ladite presse à matricer ;
(v) compression, à la fois, de ladite première poudre et de ladite seconde poudre sur ledit enrobage compressible, sur ledit noyau, pour former un comprimé compressé ; et
(vi) facultativement, enrobage dudit comprimé compressé issu de l'étape (v) avec un enrobage extérieur,
où soit ladite première poudre, soit ladite seconde poudre comprend un second médicament.
